# EUROPEAN PATENT SPECIFICATION

(11) **EP 3 638 197 B1**
(45) Date of publication and mention of the grant of the patent: **18.01.2023**
(21) Application number: 18730720.2
(22) Date of filing: 07.06.2018
(51) Int. Cl.: A61K 8/34, A61K 8/41, A61Q 5/12, A61K 8/81

(54) **HAIR CONDITIONING COMPOSITION HAVING IMPROVED RINSING PROPERTIES**
HAARKONDITIONIERENDE ZUBEREITUNG
PRÉPARATION DE CONDITIONNEMENT DES CHEVEUX

(30) Priority: 15.06.2017 EP 17176247
(43) Date of publication of application: 22.04.2020
(73) Proprietor: Unilever IP Holdings B.V., 3013 AL Rotterdam (NL); Unilever Global IP Limited, Wirral, Merseyside CH62 4ZD (GB)
(72) Inventor: COAN, Lynsey, Joanne, Bebington Wirral Merseyside CH63 3JW (GB); GILES, Colin, Christopher, David, Bebington Wirral Merseyside CH63 3JW (GB); GLENDAY, Jennifer, Amy, Sheffield S8 8TD (GB); GUTIERREZ-ABAD, Raquel, Bebington Wirral Merseyside CH63 3JW (GB); LUCK, Matias, Wirral Merseyside CH62 2EY (GB)
(74) Representative: Chisem, Janet
(86) International application number: PCT/EP2018/064957
(87) International publication number: WO 2018/228900

(56) References cited:
- WO-A1-2009/153281
- WO-A1-2014/016350
- WO-A1-2015/110510
- WO-A1-2015/110511
- WO-A2-2006/081496
- US-A1- 2009 291 058
- "Aculyn? 28 Rheology Modifier/Stabilizer A Very Efficient Thickener for a Wide Array of Personal Care Formulations", ROHM HAAS PERSONAL,, 1 February 2004 (2004-02-01), pages 1-16, XP007909941,
- Umbach: "Kosmetik" In: "Kosmetik", 1 January 1998 (1998-01-01), XP055393819, ISBN: 978-3-527-30875-0 pages 250-251,
- Anonymous: "Preservaties master sheet", , 1 January 2018 (2018-01-01), XP055498868, Retrieved from the Internet: URL:https://chemistscorner.com/cosmeticsci encetalk/uploads/editor/e7/bo2bz46w4o9a.pd f [retrieved on 2018-08-10]

## Description

### Field of the Invention

The invention relates to an improved rinse-off hair conditioning composition, that enables less water to be used during use.

### Background and Prior art

Reducing the amount of water that is used in everyday tasks and activities, such as washing of hair, reduces the energy required to process and deliver it to homes, businesses and communities. This in turn helps to reduce pollution and conserve fuel resources. Reducing the amount of water that goes to waste at home helps protect the wildlife that lives in rivers and wetlands. Thus, hair treatment products that require reduced rinsing to remove from the hair not only save consumers time and effort but can also save ecosystems, energy and water.

Technologies are known that improve rinsing properties of products from hair or hands.

WO 13/092708 (L'Oreal) discloses a cosmetic composition, especially a hair composition, comprising at least one anionic or nonionic associative polymer, at least one fixing polymer and at least one specifically defined nonionic surfactant. A styling gel conforming to this composition is purported to have improved hold of the hairstyle over time and to be easily removed from the hands and the hair with water, without shampoo or soap.

WO 15/001071 (L'Oreal) discloses a non-colouring hair composition comprising 2 - 60 wt % of at least one anionic copolymer, water-soluble inorganic salts and one or more alkaline agents. The anionic copolymer may be an anionic associative polymer, such as are capable of associating reversibly with one another or with other molecules. Numerous benefits of these compositions are disclosed, and they are said to be particularly capable of generating an adequate foam, in terms of quality and quantity, and of giving the hair satisfactory cosmetic properties, such as sheen, softness, smoothness, disentangling and suppleness, most particularly on dry hair. Further they are purported to be rinsed out faster than conventional shampoos, to have a more pronounced treating nature and to give the hair more lightness, in particular wet hair. They don't necessarily require the addition of viscosity enhancers, can afford varied textures and are better tolerated by the scalp and the eyes. Detergent compositions used on hair which are said to provide a foaming effect and good cosmetic properties, are exemplified.

Related case WO15/001072 (L'Oreal) discloses a self-foaming non-colouring hair composition comprising from 2 to 60 wt % of one or more anionic, or non-ionic associative polymers, surfactant and propellant gas.

WO 09/153281 (Unilever) discloses a hair conditioning composition comprising hydrophobically modified anionic polymer. The inclusion of a crystalline wax as a structurant is preferred and exemplified. The polymer is said to provide better rinse-off properties and an example discloses better ease of rinse in a composition comprising the polymer compared with a similar composition without the polymer.

Related case WO 09/153280 (Unilever) discloses hair conditioning compositions comprising hydrophobically modified anionic polymer, a silicone and a fatty acid to give improved deposition of silicone. Better rinse off properties are mentioned but silicone deposition is exemplified.

Another document form this field is WO 06/081496.

Compositions that are easy to rinse do not necessarily require less water to accomplish the rinse. They may, for example, require less mechanical agitation, or even no agitation, but a longer rinse time, so less effort is required but not less water. Despite the technological advances there remains a need for conditioning compositions for use on hair, that require reduced water to rinse effectively and quickly without compromising the performance of the product as a conditioner.

We have now surprisingly found that a conditioning composition that comprises a conditioning base and a hydrophobically modified anionic polymer can be used in the treatment of hair to reduce the amount of water required to rinse without reducing the conditioning benefits on the hair. We have found that when a consumer rinses conditioner from his/her hair, he/she will stop rinsing when a satisfactory constant level of smooth feel is reached (referred to herein as the "rinsed friction plateau"). The composition of the invention enables the consumer to reach his/her rinsed friction plateau sooner, when compared to a composition comprising the same ingredients but without the hydrophobically modified anionic polymer, thus causing him/her to stop rinsing and therefore consume less water. Importantly, we have found that the conditioning benefits, particularly superior slippery feel in the wet, of the composition are not comprised.

### Definition of the Invention

Accordingly, there is provided a hair conditioning composition as defined in claim 1.

In a second aspect, the invention provides a method of reducing the amount of water used to rinse hair comprising the steps of applying to hair a composition of the first aspect and rinsing the hair with water.

In a third aspect, the invention provides a use of a hydrophobically modified anionic polymer in a hair treatment composition of the first aspect, to reduce the amount of water required to rinse the composition from the hair.

In the method of the invention, preferably, the hair is rinsed with water until a constant friction is reached.

The use of the invention, is preferably to reduce the amount of water required to rinse the hair treatment composition from the hair until a constant friction is reached. Preferably, the hair treatment composition for use in the use of the invention is that of the first aspect of the invention.

### The hydrophobically modified anionic polymer

Preferably, the hydrophobically modified polymer is an acrylate or methacrylate polymer.

Preferably, the hydrophobic modification comprises alkylation. Preferably, the alkyl group comprises from 6 to 30 carbons, more preferably from 16 to 28 and most preferably from 18 to 24 carbons.

A preferred polymer is sold by Rohm & Haas under the tradename Aculyn, the most preferred of which is Aculyn 28^{™}.

The polymer is present at from 0.01 to 5 wt % and more preferably from 0.05 to 1 wt %, by total weight of the hair conditioning composition.

### The conditioning base

Compositions in accordance with the invention are preferably formulated as conditioners for the treatment of hair (typically after shampooing) and subsequent rinsing.

The composition confers a Draw Mass of from 1 to 250 g, preferably 2 to 100 g, more preferably 2 to 50 g, even more preferably 5 to 40 g and most preferably 5 to 25 g to hair treated with the conditioning composition.

Draw Mass is the mass required to draw a hair switch through a comb or brush. Thus the more tangled the hair the greater the mass required to pull the switch through the comb or brush, and the greater the level of condition of the hair, the lower the Draw Mass.

The Draw Mass is the mass required to draw a hair switch, for example of weight 1 to 20 g, length 10 to 30 cm, and width 0.5 to 5 cm through a comb or brush, as measured by first placing the hair switch onto the comb or brush, such that from 5 to 20 cm of hair is left hanging at the glued end of the switch, and then adding weights to the hanging end until the switch falls through the comb or brush.

Preferably, the hair switch is of weight 1 to 20 g, more preferably 2 to 15 g, most preferably from 5 to10 g. Preferably, the hair switch has a length of from 10 to 40 cm, more preferably from 10 to 30 cm, and a width of from 0.5 to 5 cm, more preferably from 1.5 to 4 cm.

Most preferably, the Draw Mass is the mass required to draw a hair switch, for example of weight 10 g, length 20 cm, and width 3 cm through a comb or brush, as measured by first placing the hair switch onto the comb or brush, such that from 20 cm of hair is left hanging at the glued end of the switch, and then adding weights to the hanging end until the switch falls through the comb or brush.

In one embodiment, the composition comprises a conditioning gel phase obtainable by:
forming a comelt in a first vessel comprising fatty alcohol and cationic component and 0.1 to 15 wt % water, by total weight of the comelt (A);
adding the comelt to a second vessel containing water at 50 to 60°C (B); and mixing,
wherein the temperature of the mixture of the comelt and the water in the second vessel (B) is controlled such that it is maintained at from 56 to 65°C, preferably from 58 to 62°C, more preferably 60°C, for from 5 to 60 min, preferably for from 10 to 40 min, more preferably from 15 to 30 min;
wherein the fatty alcohol has from 8 to 22 carbons;
wherein the cationic component comprises from 0.1 to 70 wt % cationic surfactant having the formula N⁺R¹R²R³R⁴, more preferably from 30 to 60 wt %, by total weight of the cationic component; and
wherein R¹, R², R³ and R⁴ are independently (C₁ to C₃₀) alkyl or benzyl.

The comelting of the fatty alcohol and the cationic surfactant forms an isotropic phase.

This means that the development of structure, i.e. the formation of the lamellar conditioning gel phase, can be controlled by the temperature and rate of mixing of the comelt and the water. The conditioning composition ultimately made using such conditioning gel phase has superior conditioning capability which is demonstrated by the reduced Draw Mass.

Preferably, the water in the second vessel is maintained at 56 to 60°C and more preferably at 57 to 59°C preferably for from 10 to 40 min, more preferably from 15 to 30 min. This temperature control during the process of preparation results in a balance of thermal energy at the point of mixing the water with the comelt. If the water is too cold then the comelt solidifies resulting in a poorly mixed system and this ultimately provides a composition of low viscosity. If the temperature of the water is too high then it is also too high at the point of mixing with the comelt and so forms vesicles. This also gives rise to lower viscosity in the conditioning composition formed with the resulting conditioning gel phase.

Preferably, the comelt comprises from 45 to 90 wt % fatty alcohol, by weight of the comelt.

Preferably, the fatty alcohol comprises from 8 to 22 carbon atoms, more preferably 16 to 22. Fatty alcohols are typically compounds containing straight chain alkyl groups. Examples of suitable fatty alcohols include cetyl alcohol, stearyl alcohol and behenyl alcohol and mixtures thereof. The use of these materials is particularly preferable.

The level of fatty alcohol in the conditioner of the invention (not just the conditioning gel phase) will generally range from 0.01 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Preferably, the comelt comprises from 10 to 40 wt % cationic component, by total weight of the comelt.

In a most preferred embodiment the conditioning composition is made by first preparing a conditioning gel phase which is formed by adding cationic surfactants to fatty alcohol and stirring at 85°C.

This mixture is gradually added to water, containing other ingredients, typically at 55°C, but at a temperature tailored to the composition to ensure that the mixture temperature is 60°C, this temperature maintained by external heating if required, preferably for from 10 to 40 min, more preferably from 15 to 30 min;
and stirred.

The mixture is cooled towards ambient by adding more water, and other ambient temperature ingredients, and use of external cooling if required, and stirred.

Remaining components of the conditioning composition may then be added.

In an alternative embodiment the conditioning composition of the invention is obtainable by first forming a conditioning gel phase by:
forming a comelt in a first vessel comprising fatty alcohol and cationic component and 0.1 to 15 wt % water, by total weight of the comelt, independently adding the comelt and
water to a mixing vessel and mixing, wherein the temperature of the mixture of the comelt and the water is maintained at from 56 to 65°C, preferably from 58 to 62°C, more preferably 60°C when in the mixing vessel,
wherein the fatty alcohol comprises from 8 to 22 carbons,
wherein the cationic component comprises from 0.1 to 70 wt % cationic surfactants have the formula N⁺R¹R²R³R⁴, more preferably from 30 to 60 wt % by total weight of the cationic component, and
wherein R¹, R², R³ and R⁴ are independently (C₁ to C₃₀) alkyl or benzyl.

Preferably, the comelt and aqueous dispersion are mixed for from 10 to 40 min, more preferably from 15 to 30 min.

Preferably, the process is a continuous process.

The comelt of the invention forms an isotropic phase which means the development of structure, i.e. the formation of the lamellar conditioning gel phase,
can be controlled. In this process the temperature of the mixture of comelt and water is controlled by modifying the temperature of water added to the mix. Water may be added in one go or it may be staged. Typically, a first water vessel is maintained at around 40°C and is pumped into the mixing vessel while a second water vessel is maintained at a sufficient temperature to modify the temperature of the mixture of water with comelt such that it falls within the required range, i.e. from 56 to 65°C, preferably from 58 to 62°C, more preferably 60°C in the mixing vessel.

The conditioning composition ultimately made using such conditioning gel phase exhibits improved conditioning characteristics which are not observed when the conditioning gel phase is formed in the comelt.

As discussed above, the improvement thus resides in the balance of thermal energy at the point of mixing the water with the comelt. If too cold then one ends up with a poorly mixed system due to the tendency for the comelt to solidify and this ultimately provides a composition of low viscosity. If the temperature of the mix vesicles form. This also gives rise to lower viscosity in the conditioning composition formed in the long run.

Preferably, the comelt comprises from 45 to 90 wt % fatty alcohol, by weight of the comelt.

Preferably, the fatty alcohol comprises from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is particularly preferable.

The level of fatty alcohol in the conditioner of the invention (not just the conditioning gel phase) will generally range from 0.01 to 10%, preferably from 0.1% to 8%, more preferably from 0.2 % to 7 %, most preferably from 0.3 % to 6 % by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Preferably, the comelt comprises from 10-40% wt. of the comelt cationic surfactant, by adding cationic surfactants to fatty alcohol and stirring at 85°C.

Inject this mixture into a flowing stream of water, containing other ingredients, the temperature of the water varied to ensure this mixture has a temperature of 60°C and mix.

Cool this stream towards ambient by injection into a second water stream and mix.

In an alternative embodiment the composition comprises a conditioning gel phase obtainable by:
forming an aqueous isotropic solution of cationic component;
mixing the aqueous isotropic solution of cationic surfactant with molten fatty alcohol,
wherein the temperature during mixing the fatty alcohol with the isotropic cationic surfactant solution is maintained from 55°C to 65°C, preferably for 10 to 60 minutes, more preferably for 20 to 40 minutes, most preferably 15 to 30 minutes and wherein the fatty alcohol has from 8 to 22 carbons.

A conditioning composition made using a conditioning gel phase of the invention has been shown to be superior to compositions made by standard processes where the materials are mixed in water at around 70°C. The superior conditioning manifests itself in superior conditioner thickness (despite having lower solids levels) and next day clean feel and conditioning benefits. These are surprising since it would be expected that superior conditioning products usually leave the hair lank and greasy the following day sue to excessive deposition of solids.

Preferably, the temperature of the mixture of the aqueous isotropic solution and fatty alcohol is maintained at from 55°C to 65°C, preferably for 10 to 60 minutes, more preferably for 20 to 40 minutes, most preferably 15 to 30 minutes.

Preferably, the molten fatty alcohol is added to the aqueous isotropic solution of cationic surfactant.

In this process the temperature of the mixture is controlled by modifying the temperature/rate of the mixture of the fatty alcohol and the cationic surfactant solution. The temperature needs to be carefully controlled in order to achieve the right conditioning gel phase structure. The improvement thus resides in the balance of thermal energy at the point of mixing the fatty alcohol with the isotropic mixture.

After formation of the gel phase further water and additional ingredients may be added in one go or it may be staged. Preferably the gel phase is cooled prior to addition of the water.

The conditioning composition ultimately made using such conditioning gel phase has improved conditioning capabilities.

Preferably, the temperature of the mixture of the fatty alcohol and aqueous isotropic solution is maintained at from 58°C to 62°C; most preferably at 60°C, preferably for 10 to 60 minutes, more preferably for 20 to 40 minutes, most preferably 15 to 30 minutes.

Preferably, and prior to addition to the isotropic mixture, the fatty alcohol is maintained at a temperature sufficient to maintain the fatty alcohol in a liquid phase. Preferably the fatty alcohol is maintained at from 80°C to 85°C.

Preferably, the resulting conditioning gel phase is mixed with a mixer having a rotor tip speed of 10-34, preferably from 21-27 and especially preferably 24 ms-1.

Preferably, the fatty alcohol comprises from 8 to 22 carbon atoms, more preferably 16 to 22. Fatty alcohols are typically compounds containing straight chain alkyl groups. Examples of preferred fatty alcohols include cetyl alcohol, stearyl alcohol and mixtures thereof.

The level of fatty alcohol in the conditioner of the invention (not just the conditioning gel phase) will generally range from 0.01 to 10%, preferably from
0.1 % to 8%, more preferably from 0.2 % to 7 %, most preferably from 0.3 % to
6% by weight of the composition. The weight ratio of cationic surfactant to fatty alcohol is suitably from 1:1 to 1:10, preferably from 1:1.5 to 1:8, optimally from
1:2 to 1:5. If the weight ratio of cationic surfactant to fatty alcohol is too high, this can lead to eye irritancy from the composition. If it is too low, it can make the hair feel squeaky for some consumers.

Further conditioning composition ingredients are added as necessary to form the conditioning composition.
In an alternative embodiment the conditioning composition comprises a conditioning gel phase obtainable by forming an aqueous dispersion of fatty alcohol and amidoamine;
adding a cationic surfactant to the aqueous dispersion and mixing; and
neutralising the amidoamine,
wherein the temperature of the mixture of cationic surfactant in the aqueous dispersion is maintained at from 56°C to 67°C, preferably for 10 to 60 minutes, more preferably for 20 to 40 minutes, most preferably 15 to 30 minutes.

Conditioning compositions made with the conditioning gel phase of the invention have improved conditioning performance. More specifically, the conditioning compositions made using the conditioning gel phase of the invention are thicker, even when using a lower level of solids, and provide improved clean feel the following day. This is surprising since one usually associates improved conditioning with increased deposition of solids which results on greasiness and heaviness the next day. To provide the opposite is an unmet consumer need.

Preferably, the temperature of the aqueous dispersion is maintained above the melting temperature of the fatty alcohol, preferably at least 5°C higher than the melting point of the fatty alcohol.

Preferably, the aqueous dispersion is formed by adding fatty alcohol to water heated and maintained at least the melting point of the fatty alcohol and preferably at least 5°C above the melting point of the fatty alcohol. Preferably, the aqueous dispersion is maintained at a melting point sufficient to maintain the fatty alcohol in a liquid phase.

Preferably, the temperature of the mixture of the aqueous dispersion is controlled such that it is maintained from 56-67°C, preferably from 58-65°C, more preferably 63°C, preferably for 10 to 60 minutes, more preferably for 20 to 40 minutes, most preferably 15 to 30 minutes.

Preferably, the temperature of the mixture of the aqueous dispersion and the cationic surfactant is maintained at from 56°C to 67°C. More preferably, the temperature of the mix of the aqueous dispersion and the cationic surfactant is maintained at from 58°C to 65°C; most preferably at 63°C.

Controlling the temperature of the mixture of fatty alcohol and the cationic surfactant means controlling the formation of gel structure. In this process the temperature of the mixture of comelt and water is controlled by modifying the temperature/rate of the cationic surfactant to the fatty alcohol and an amidoamine surfactant aqueous mix. If too cold or too hot then a system having a mixture of structures results and this has poorer conditioning capability.

After formation of the gel phase further water and additional ingredients may be added in one go or it may be staged.

Preferably, the process is a batch process.

Preferably the mixing of the cationic surfactant with the aqueous dispersion is monitored by measurement of viscosity, such that when the viscosity change plateaus, the required degree association has occurred and then the amidoamine is neutralised. Typically, this mixing of the cationic surfactant and aqueous dispersion takes from 20 to 60 minutes. The conditioning composition ultimately made using such conditioning gel phase has improved conditioning performance compared with an identical conditioning composition made with an identical formulation made using a standard process.

Preferably, the process comprises passing the contents of the mixture vessel through a mixer with rotor tip speed of 10-34, preferably from 21-27 and especially preferably 24 ms-1.

Preferably the aqueous dispersion comprises from 25 wt.% to 50 wt.%, more preferably from 35 to 45 wt.% of the total dispersion water.

Preferably the aqueous dispersion comprises from 4 to 20 wt.% of the total dispersion fatty alcohol.

Preferably the aqueous dispersion comprises from 1 to 5 wt.% of the total dispersion amidoamine.

Preferably the neutraliser added to the aqueous dispersion and cationic surfactant comprises sufficient neutraliser to neutralise at least 90 wt% of the cationic surfactant, more preferably at least 95 % of the cationic surfactant, most preferably at least 99 % of the cationic surfactant.

Preferably, the fatty alcohol comprises from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is particularly preferable.

The level of fatty alcohol in the conditioner of the invention (not just the conditioning gel phase) will generally range from 0.01 to 10%, preferably from
0.1 % to 8%, more preferably from 0.2 % to 7 %, most preferably from 0.3 % to
6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Preferably, the conditioning gel phase is obtainable by adding a stearylamidopropyl dimethylamine and fatty alcohol to water at 60°C, maintain temperature by use of external heating, and stir.

Add a cationic surfactant, typically behentrimonium chloride, to this mixture, maintain temperature at 58-65°C by use of external heating or cooling, and stir.

Add lactic acid to protonate stearylamidopropyl dimethylamine, maintain temperature at 58-65°C by use of external heating or cooling, and stir.

Cool this towards ambient by adding more water, and other ambient temperature ingredients, and use of external cooling if required, and stir.

Further ingredients are then added to form a conditioning composition.

Suitable conditioning surfactants include those selected from cationic surfactants, used singly or in admixture. Preferably, the cationic surfactants have the formula N⁺R¹R²R³R⁴ wherein R¹, R², R³ and R⁴ are independently (C₁ to C₃₀) alkyl or benzyl. Preferably, one, two or three of R¹, R², R³ and R⁴ are independently
(C₄ to C₃₀) alkyl and the other R¹, R², R³ and R⁴ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester
(-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride,
hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Preferably, the cationic surfactant of the comelt comprises from 0-70% cationic surfactants have the formula N⁺R¹R²R³R⁴ as described above, more preferably from 30-60% wt. cationic surfactant.

Another example of a class of suitable cationic surfactants for use in the invention, either alone or together with one or more other cationic surfactants, is a combination of (i) and (ii) below:
(i) an amidoamine corresponding to the general formula (I):

   (I) R1CONH(CH2)mN(R2)R3

   in which R¹ is a hydrocarbyl chain having 10 or more carbon atoms, R² and R³ are independently selected from hydrocarbyl chains of from 1 to 10 carbon atoms, and m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyl-diethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyl-dimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include:
stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a
tradename INCROMINE BB available from Croda (North Humberside, England), and various amidoamines with tradenames SCHERCODINE series available from Scher (Clifton New Jersey, USA).

Acid may be any organic or mineral acid, which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate more than
95 mole% (293 K) of the amidoamine present.

Should an amidoamine of the type described herein be present then the corresponding acid component will not be present in the comelt. Instead it will be present in the water. Preferably, the water comprises protonating component at from 0.01 to 3% wt. Accordingly, where the invention requires from 10-40% wt. comelt cationic surfactant, the cationic surfactant may comprise amidoamine which is not protonated, i.e. it will not be cationic charged but will become protonated when added to the water and hence the protonating material contained therein.

Preferably, the cationic surfactant of the comelt comprises from 0-70% amidoamine corresponding to formula (I), more preferably from 30-60% wt. by total weight of the cationic surfactant.

In conditioning compositions of the invention (not merely the conditioning gel phase), the level of cationic surfactant will generally range from 0.01 % to 10%, more preferably 0.05 % to 7.5%, most preferably 0.1 % to 5% by weight of the composition.

Preferably, where a comelt is used, the comelt is maintained at a melting point sufficient to maintain the fatty alcohol in a liquid phase. Preferably, the comelt is maintained at from 80-85C.

Preferably, the temperature of the mixture of the comelt and the water is controlled such that it is maintained from 56-65C, prefer from 58-62C, more preferably 60C during mixing.

Preferably, the contents of the mixture vessel passed through a mixer with rotor tip speed of 10-34, preferably from 21-27 and especially preferably 24 ms-1.

In a further aspect there is provided a process for manufacturing a conditioning composition by forming a conditioning gel phase obtained as described above and then adding any remaining ingredients. Typical remaining ingredients include fragrances, silicones, fibre actives or other benefit agents.

Preferably, the conditioning composition is passed through a mixer with rotor tip speed of 10-34, preferably from 21-27 and especially preferably 24 ms-1 one more time after the remaining ingredients have been added.

The conditioning phase has a lamellar structure, which is formed during the preparation methods described above.

### Silicones

Conditioning compositions of the invention or using conditioning gel phases of the invention also deposit silicone better than conventionally made conditioning compositions.

Accordingly, the compositions of the invention can contain, emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of crosslinking, as are described for example in WO 96/31188.

The viscosity of the emulsified silicone itself (not the emulsion or the final hair conditioning composition) is typically at least 10,000 cst at 25oC the viscosity of the silicone itself is preferably at least 60,000 cst, most preferably at least 500,000 cst, ideally at least 1,000,000 cst. Preferably the viscosity does not exceed 109 cst for ease of formulation.

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of 0.15 micron are generally termed microemulsions.

Emulsified silicones for use in the conditioner compositions of the invention will typically have an size in the composition of less than 30, preferably less than 20, more preferably less than 15. Preferably the average silicone droplet is greater than 0.5 micron, more preferably greater than 1 micron, ideally from 2 to 8 micron.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions

DC2-7224, DC2-8467, DC2-8177 and DC2-8154 (all ex Dow Corning).

The total amount of silicone is preferably from 0.01 wt% to 10 %wt of the total composition more preferably from 0.1 wt% to 5 wt%, most preferably 0.5 wt% to 3 wt% is a suitable level.

### Further Ingredients

The composition according to the invention may comprise any of a number of ingredients which are common to conditioning compositions

Other ingredients may include viscosity modifiers, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally, these optional ingredients are included individually at a level of up to about 5% by weight of the total composition.

Preferably, compositions of this invention also contain adjuvants suitable for hair care. Generally, such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
   - free fatty acids, for cuticle repair and damage prevention. Examples are branched chain fatty acids such as 18-methyleicosanoic acid and other homologues of this series, straight chain fatty acids such as stearic, myristic and palmitic acids, and
   unsaturated fatty acids such as oleic acid, linoleic acid, linolenic acid and arachidonic acid. A preferred fatty acid is oleic acid. The fatty acids may be added singly, as mixtures, or in the form of blends derived from extracts of, e.g. lanolin.

Mixtures of any of the above active ingredients may also be used.

In a second aspect there is provided a method for the manufacture of a conditioning composition according to the first aspect. The method comprising forming a conditioning gel phase which comprises a cationic surfactant and a fatty material and, separately forming a solution of the hydrophobically modified polymer, optionally with a cationic surfactant, which, if present, is added to the water first.

The two mixtures are then added to one another before the remaining ingredients are added to form the conditioning composition.

Preferably, the extra ingredients include perfumes, thickeners, preservatives, colours and conditioning silicones.

### Example 1: Composition 1 in accordance with the invention and Comparative Compositions A. B and C

The following compositions were prepared:

**Table 1: Compositions of Composition 1 in accordance with the invention, and Comparative Compositions A, B and C.**

| Material (based on 100 % active) | Amount (wt %) | | | |
|---|---|---|---|---|
| | A | B | C | 1 |
| Behenyl Trimethyl Ammonium Chloride | 1 | 1 | 1 | 1 |
| Stearamidopropyldimethylamine | 1 | 1 | 1 | 1 |
| Lactic acid | 0.32 | 0.32 | 0.32 | 0.32 |
| Ceterearyl Alcohol | 4 | 4 | 4 | 4 |
| Amodimethicone/Dimethicone | 1.5 | 1.5 | 1.5 | 1.5 |
| Acrylates/Beheneth-25 Methacrylate Coploymer (Aculyn 28) | 0 | 0.1 | 0 | 0.1 |
| Fragrance/preservatives/water | to 100 | to 100 | to 100 | to 100 |

Conditioners A and B were prepared using the following method:
1. Water was added to a suitable vessel, lactic acid and the copolymer were added, and the vessel heated to 80 °C.
2. Cetearyl alcohol was then added to the formulation along with tertiary amine salt (stearamidopropyldimethylamine).
3. At 80°C the Behenyl Trimethyl Ammonium Chloride (BTAC) was added and the resultant mixture mixed until opaque and thick.
4. The heat was then turned off and the quench water was added.
5. The mixture was then cooled to below 40°C the rest of the materials, including fragrance, were added.
6. Finally the formulation was mixed at high shear on a Silverson mixer at 5000rpm for 5 minutes.

Conditioners 1 and C were prepared using the following method:
1. Water was added to a suitable vessel, lactic acid and the copolymer were added, and the vessel heated to 63°C.
2. Cetearyl alcohol was then added to the formulation along with the tertiary amine salt (stearamidopropyldimethylamine). These were allowed to melt.
3. When the temperature of the water phase had returned to its original temperature, Behenyl Trimethyl Ammonium Chloride (BTAC) was added to the formulation and the resultant mixture mixed for 30 minutes.
4. The heat was then turned off and the quench added slowly.
5. The mixture was then cooled to below 40°C and the rest of the materials, including fragrance, were added and the mixture thoroughly mixed using a Silverson mixer at 5000rpm for 5 minutes.

### Example 2: Treatment of hair with compositions A-C and 1

The hair used was dark brown European hair, in switches of 2.5 g weight and 6 inch length.

Hair was first treated with a cleansing shampoo using the following method:-
The hair fibres were held under running water for 30 seconds, shampoo applied at a dose of 0.1 ml of shampoo per 1g of hair and rubbed into the hair for 30 seconds. Excess lather was removed by holding under running water for 30 seconds and the shampoo stage repeated. The hair was rinsed under running water for 1 minute.

The wet hair was then treated with Conditioner A-C or 1 using the following method:-
The conditioner was applied, pre-diluted at 20, 40, 80 and 160 parts water, with a dosage of 1ml of conditioner solution per 1g hair, (2.5ml in total) and combed through the hair for 1 min. The hair was then securely mounted into the TA machine and friction analysis performed as below.

### Example 3: Friction measurement of hair treated with Compositions A and 1

Friction was measured using the apparatus and method of the invention as follows: Friction was measured using a TA.XT2i Texture Analyser supplied by Stable Micro Systems, Surrey, UK, and a friction probe in the form of a stainless steel cylinder, which was coated with rubber material and fitted with a weight. The friction probe had surfactant on its outer (contact) surface. The load on the friction contact was approximately 138 g. When in use, an area of contact between the outer surface of the friction probe and the hair of approximately 1.0 cm² was achieved.

Surfactant was added to the probe as follows:
The probe was first washed with an aqueous composition of Sodium Lauryl Ether Sulphate (SLES) at a concentration of 14 wt %, by weight of the total aqueous surfactant composition, and rinsed with water. The probe was then soaked in a dilute solution of SLES having a concentration of 14 ppm, for 2 minutes, and then dried for 2 hours.

The methodology used to assess the friction properties of hair treated with Conditioners A-C and 1 was as follows:
A switch of hair was securely mounted onto the texture analyser, the hair fibres being aligned by combing before being secured in a flat configuration. The hair was immersed in the water bath. The friction probe was placed onto the hair and moved along the hair at a speed of 10 mms⁻¹ to measure the friction between the probe and the hair. The measurement was repeated 30 times.

The friction values reported below are of friction hysteresis in units of gfmm, obtained by integrating the total measured friction force over the total distance travelled by the probe, with and against cuticle.

The conditioner was pre-diluted at 20, 40, 80 and 160 parts water and used to treat hair using the method described above. Friction measurements were then performed. Immersed friction measured on hair switches treated with Conditioners A-C and 1 are given in the Table below.

### Example 4: Friction measurement of hair treated with Compositions A-C and 1

**Table 2: Friction (gfmm) of hair treated with Comparative A and Composition 1 in accordance with the invention, after dilution levels of 20, 40, 80 and 160.**

| Dilution | A | B | C | 1 | significance |
|---|---|---|---|---|---|
| 20 | 102 | 93 | 88.6 | 85.6 | ns |
| 40 | 86.7 | 111.9 | 92.2 | 121.9 | >95% |
| 80 | 92.2 | 128.3 | 99.7 | 135.6 | >95% |
| 160 | 143.7 | 124 | 135.9 | 139.6 | >95% |

It will be seen that a rinsed friction plateau is reached for hair treated with the composition of the invention sooner than with the comparative compositions.

### Example 5: Composition 2 in accordance with the invention and Comparative Composition D

The following compositions were prepared using the same method as described above for Examples 1 and C

**Table 3: Compositions of Composition 2 in accordance with the invention, and Comparative Composition D.**

| Material (based on 100 % active) | Amount (wt %) | |
|---|---|---|
| | D | 2 |
| Behenyl Trimethyl Ammonium Chloride | 1.37 | 1.37 |
| Stearamidopropyldimethylamine | 0.3 | 0.3 |
| Lactic acid | 0.32 | 0.32 |
| Ceterearyl Alcohol | 3.1 | 3.1 |
| Amodimethicone/Dimethicone | 1.4 | 1.4 |
| Acrylates/Beheneth-25 Methacrylate Coploymer (Aculyn 28) | 0 | 0.25 |
| Fragrance/preservatives/water | to 100 | to 100 |

### Example 6: Viscosity of Compositions 2 and D under dilution

When a conditioning gel phase composition is applied to hair during a wash/care process, the gel phase is deposited onto the hair surface. When the deposited gel phase comes into contact with water (during a rinse step), the structure of the gel phase must be broken up in order for it to be efficiently removed from the hair. The greater the disruption to the gel phase, the easier and faster it is removed and, *ipso facto,* the less water is required to complete the rinse. The disruption to the composition gel phase can be indicated by a reduction in its viscosity upon dilution with water.

For any given quantity of water, the extent of viscosity reduction indicates how quickly and easily it will be removed from the hair. This correlates with the amount of water used to rinse a conditioning composition from hair.

In the following examples, viscosity measurements were carried out on aqueous dilutions of the neat composition (Compositions 2 and D).

Samples were measured using a Brookfield viscometer with a T-A spindle as well as RV5.

The samples were prepared as 150 g dilutions as follows:
Composition (for example 75 g for a 1 in 2 dilution) was added to a beaker. Water (75 g for a 1 in 2 dilution) was then added in small amounts with mixing until homogeneous.

The sample was left to equilibrate for one hour before measurement with the Brookfield viscometer.

In this way, a series of dilutions were prepared (ensuring consistent mixing and speed of water addition throughout).

The samples were measured using the Brookfield RVDV-II+ viscometer with the following conditions: T-A bar spindle: 0.5rpm; 60s measurement; 5 replicates per sample.

The results are given in the following table:

**Table 4: Viscosity of Composition 2 (in accordance with the invention) and Comparative Composition D.**

| | **Viscosity** / **cP** | | | **Normalised data** | |
|---|---|---|---|---|---|
| **Dilution** | **D** | **2** | | **D** | **2** |
| Neat | 398,400 | 656,800 | | 500,000 | 500,000 |
| 1 in 1.25 | 141,200 | 143,200 | | 177208.84 | 109013.40 |
| 1 in 1.5 | 113,600 | 72,000 | | 142570.28 | 54811.21 |
| 1 in 1.75 | 67,600 | 33,200 | | 84839.36 | 25274.06 |
| 1 in 2 | 43,200 | 13,600 | | 54216.87 | 10353.23 |
| 1 in 3 | 9,600 | 4,000 | | 12048.19 | 3045.07 |
| 1 in 4 | 6,800 | 0 | | 8534.14 | 0.00 |

It will be seen that the viscosity of the composition in accordance with the invention drops dramatically faster than that of the comparative example, ensuring saving of water during a rinse step of a conditioning process.

## Claims

1. A hair conditioning composition comprising:
a) a conditioning gel phase comprising, by total weight of the hair conditioning composition,
i) from 0.4 to 8 wt % of fatty alcohol having from 8 to 22 carbons,
ii) from 0.1 to 2 wt % of cationic surfactant,
b) a hydrophobically modified anionic polymer, which comprises hydrophobic modification that comprises an alkyl group comprising from 6 to 30 carbon atoms; and
c) water,
wherein the composition confers a Draw Mass of from 1 to 250 g to hair treated with the conditioning composition, wherein Draw Mass is the mass required to draw a hair switch of weight 10 g, length 20 cm, and width 3 cm through a comb or brush, as measured by first placing the hair switch onto the comb or brush, such that from 20 cm of hair is left hanging at the glued end of the switch, and then adding weights to the hanging end until the switch falls through the comb or brush;
and wherein the conditioning gel phase is obtainable from a method selected from the following:-
i) forming a comelt in a first vessel comprising fatty alcohol and cationic component and 0.1 to 15 wt % water, by total weight of the comelt (A); adding the comelt to a second vessel containing water at 50 to 60°C (B); and mixing,
wherein the temperature of the mixture of the comelt and the water in the second vessel (B) is controlled such that it is maintained at from 56 to 65°C, preferably from 58 to 62°C, more preferably 60°C, for from 5 to 60 min, preferably for from 10 to 40 min, more preferably from 15 to 30 min;
wherein the fatty alcohol has from 8 to 22 carbons;
wherein the cationic component comprises from 0.1 to 70 wt % cationic surfactant having the formula N⁺R¹R²R³R⁴, more preferably from 30 to 60 wt %, by total weight of the cationic component; and
wherein R¹, R², R³ and R⁴ are independently (C₁ to C₃₀) alkyl or benzyl;
ii) forming a comelt in a first vessel comprising fatty alcohol and cationic component and 0.1 to 15 wt % water, by total weight of the comelt, independently adding the comelt and water to a mixing vessel and mixing, wherein the temperature of the mixture of the comelt and the water is maintained at from 56 to 65°C, preferably from 58 to 62°C, more preferably 60°C when in the mixing vessel, for from 5 to 60 min, preferably for from 10 to 40 min, more preferably from 15 to 30 min;
wherein the fatty alcohol comprises from 8 to 22 carbons,
wherein the cationic component comprises from 0.1 to 70 wt % cationic surfactants have the formula N⁺R¹R²R³R⁴, more preferably from 30 to 60 wt % by total weight of the cationic component, and
wherein R¹, R², R³ and R⁴ are independently (C₁ to C₃₀) alkyl or benzyl;
iii) forming an aqueous isotropic solution of cationic component;
mixing the aqueous isotropic solution of cationic surfactant with molten fatty alcohol,
wherein the temperature during mixing the fatty alcohol with the isotropic cationic surfactant solution is maintained from 55°C to 65°C, preferably for 10 to 60 minutes, more preferably for 20 to 40 minutes, most preferably 15 to 30 minutes and wherein the fatty alcohol has from 8 to 22 carbons;
iv) forming an aqueous dispersion of fatty alcohol and an amidoamine;
adding a cationic surfactant to the aqueous dispersion and mixing; and neutralising the amidoamine,
wherein the temperature of the mixture of cationic surfactant in the aqueous dispersion is maintained at from 56°C to 67°C, preferably for 10 to 60 minutes, more preferably for 20 to 40 minutes, most preferably 15 to 30 minutes.

2. Composition according to claim 1 wherein the polymer is an acrylate polymer.

3. Composition according to any preceding claim wherein the polymer is a methacrylate polymer.

4. Composition according to any preceding claim, wherein the alkyl group comprises from 16 to 28 carbons.

5. Composition according to any preceding claim comprising from 0.01 to 5% wt. polymer.

6. Composition according to any preceding claim wherein the cationic surfactant has the formula N⁺(R¹)(R²)(R³)(R⁴), wherein R¹, R², R³ and R⁴ are independently (C₁ to C₃₀) alkyl or benzyl.

7. Composition according to claim 6 wherein one, two or three of R¹, R², R³ and R⁴ are independently (C₄ to C₃₀) alkyl and the other R¹, R², R³ and R⁴ group or groups are (C₁-C₆) alkyl or benzyl.

8. Composition according to any preceding claim wherein the cationic conditioning surfactant is selected from the group consisting of cetyltrimethylammonium chloride, behenyltrimethylammonium chloride and mixtures thereof.

9. A method of saving water during a conditioning process, comprising the steps of applying to hair a composition comprising
a) a conditioning gel phase comprising, by total weight of the hair conditioning composition,
i) from 0.4 to 8 wt % of fatty alcohol having from 8 to 22 carbons,
ii) from 0.1 to 2 wt % of cationic surfactant,
b) a hydrophobically modified anionic polymer; and
c) water,
wherein the composition confers a Draw Mass of from 1 to 250 g to hair treated with the conditioning composition wherein Draw Mass is the mass required to draw a hair switch, for example of weight 10 g, length 20 cm, and width 3 cm through a comb or brush, as measured by first placing the hair switch onto the comb or brush, such that from 20 cm of hair is left hanging at the glued end of the switch, and then adding weights to the hanging end until the switch falls through the comb or brush;
and wherein the conditioning gel phase is obtainable from a method selected from the following:-
i) forming a comelt in a first vessel comprising fatty alcohol and cationic component and 0.1 to 15 wt % water, by total weight of the comelt (A); adding the comelt to a second vessel containing water at 50 to 60°C (B); and mixing,
wherein the temperature of the mixture of the comelt and the water in the second vessel (B) is controlled such that it is maintained at from 56 to 65°C, preferably from 58 to 62°C, more preferably 60°C, for from 5 to 60 min, preferably for from 10 to 40 min, more preferably from 15 to 30 min;
wherein the fatty alcohol has from 8 to 22 carbons;
wherein the cationic component comprises from 0.1 to 70 wt % cationic surfactant having the formula N⁺R¹R²R³R⁴, more preferably from 30 to 60 wt %, by total weight of the cationic component; and
wherein R¹, R², R³ and R⁴ are independently (C₁ to C₃₀) alkyl or benzyl;
ii) forming a comelt in a first vessel comprising fatty alcohol and cationic component and 0.1 to 15 wt % water, by total weight of the comelt, independently adding the comelt and water to a mixing vessel and mixing, wherein the temperature of the mixture of the comelt and the water is maintained at from 56 to 65°C, preferably from 58 to 62°C, more preferably 60°C when in the mixing vessel, for from 5 to 60 min, preferably for from 10 to 40 min, more preferably from 15 to 30 min;
wherein the fatty alcohol comprises from 8 to 22 carbons,
wherein the cationic component comprises from 0.1 to 70 wt % cationic surfactants have the formula N⁺R¹R²R³R⁴, more preferably from 30 to 60 wt % by total weight of the cationic component, and
wherein R¹, R², R³ and R⁴ are independently (C₁ to C₃₀) alkyl or benzyl;
iii) forming an aqueous isotropic solution of cationic component;
mixing the aqueous isotropic solution of cationic surfactant with molten fatty alcohol,
wherein the temperature during mixing the fatty alcohol with the isotropic cationic surfactant solution is maintained from 55°C to 65°C, preferably for 10 to 60 minutes, more preferably for 20 to 40 minutes, most preferably 15 to 30 minutes and wherein the fatty alcohol has from 8 to 22 carbons;
iv) forming an aqueous dispersion of fatty alcohol and an amidoamine;
adding a cationic surfactant to the aqueous dispersion and mixing; and neutralising the amidoamine,
wherein the temperature of the mixture of cationic surfactant in the aqueous dispersion is maintained at from 56°C to 67°C, preferably for 10 to 60 minutes, more preferably for 20 to 40 minutes, most preferably 15 to 30 minutes;
and rinsing the hair, compared with a comparative composition that differs from the composition in that it comprises no hydrophobically modified anionic polymer.

10. A method as claimed in claim 9, wherein the composition is as defined in any one of claims 2 to 8.

11. A method of as claimed in claimed in claim 9, wherein the hair is rinsed with water until a constant friction is reached as measured using a Texture Analyser.

12. Use of a hydrophobically modified anionic polymer in a hair treatment composition, as defined by any one of claims 1 to 8, to save water during a rinse step of a conditioning process compared with a comparative composition that differs from the composition as defined in claims 1 to 7 in that it comprises no hydrophobically modified anionic polymer.

13. Use as claimed in claim 12, where the hair is rinsed until a constant friction is reached as measured using a Texture Analyser.

## Patentansprüche

1. Haarkonditionierende Zusammensetzung, umfassend:
a) eine konditionierende Gelphase, umfassend, bezogen auf das Gesamtgewicht der haarkonditionierenden Zusammensetzung,
i) 0,4 bis 8 Gew.-% Fettalkohol mit 8 bis 22 Kohlenstoffatomen,
ii) 0,1 bis 2 Gew.-% kationisches Tensid,
b) ein hydrophob modifiziertes anionisches Polymer, welches eine hydrophobe Modifikation umfasst, die eine Alkylgruppe umfasst, umfassend 6 bis 30 Kohlenstoffatome; und
c) Wasser;
wobei die Zusammensetzung dem mit der konditionierenden Zusammensetzung behandelten Haar eine Ziehmasse von 1 bis 250 g verleiht, wobei die Ziehmasse die Masse darstellt, die erforderlich ist, um eine Haartresse eines Gewichts von 10 g, einer Länge von 20 cm und einer Breite von 3 cm durch einen Kamm oder eine Bürste zu ziehen, gemessen durch ein erstes Platzieren der Haartresse auf dem Kamm oder der Bürste, so dass 20 cm des Haars an dem verklebten Ende der Tresse hängen bleiben, und dann Gewichtszugaben auf das hängende Ende, bis die Tresse durch den Kamm oder die Bürste fällt;
und wobei die konditionierende Gelphase nach einem Verfahren erhältlich ist, ausgewählt aus Folgendem:
i) Bilden einer Co-Schmelze in einem ersten Gefäß, umfassend Fettalkohol und kationische Komponente und 0,1 bis 15 Gew.-% Wasser, bezogen auf das Gesamtgewicht der Co-Schmelze (A); Zugeben der Co-Schmelze in ein zweites Gefäß, enthaltend Wasser, bei 50 bis 60°C (B); und Mischen,
wobei die Temperatur der Mischung der Co-Schmelze und des Wassers in dem zweiten Gefäß (B) derartig kontrolliert wird, dass sie bei 56 bis 65°C, vorzugsweise bei 58 bis 62°C, bevorzugter bei 60°C, 5 bis 60 min lang, vorzugsweise 10 bis 40 min lang, bevorzugter 15 bis 30 min lang, gehalten wird; wobei der Fettalkohol 8 bis 22 Kohlenstoffatome aufweist;
wobei die kationische Komponente 0,1 bis 70 Gew.-% kationisches Tensid der Formel N⁺R¹R²R³R⁴ umfasst, bevorzugter 30 bis 60 Gew.-%, bezogen auf das Gesamtgewicht der kationischen Komponente; und
wobei R¹, R², R³ und R⁴ unabhängig voneinander (C₁- bis C₃₀-) Alkyl oder Benzyl sind;
ii) Ausbilden einer Co-Schmelze in einem ersten Gefäß, umfassend Fettalkohol und kationische Komponente und 0,1 bis 15 Gew.-% Wasser, bezogen auf das Gesamtgewicht der Co-Schmelze, unabhängig voneinander Zugeben der Co-Schmelze und des Wassers in ein Mischgefäß und Mischen,
wobei die Temperatur der Mischung der Co-Schmelze und des Wassers bei 56 bis 65°C, vorzugsweise bei 58 bis 62°C, bevorzugter bei 60°C, wenn in dem Mischgefäß, 5 bis 60 min, vorzugsweise 10 bis 40 min, bevorzugter 15 bis 30 min lang gehalten wird;
wobei der Fettalkohol 8 bis 22 Kohlenstoffatome aufweist,
wobei die kationische Komponente 0,1 bis 70 Gew.-% kationische Tenside der Formel N⁺R¹R²R³R⁴ umfasst, bevorzugter 30 bis 60 Gew.-%, bezogen auf das Gesamtgewicht der kationischen Komponente, und
wobei R¹, R², R³ und R⁴ unabhängig voneinander (C₁- bis C₃₀-) Alkyl oder Benzyl darstellen;
iii) Ausbilden einer wässrigen isotropen Lösung der kationischen Komponente;
Mischen der wässrigen isotropen Lösung des kationischen Tensids mit geschmolzenem Fettalkohol,
wobei die Temperatur während des Mischens des Fettalkohols mit der isotropen kationischen Tensid-Lösung bei 55°C bis 65°C, vorzugsweise 10 bis 60 Minuten, bevorzugter 20 bis 40 Minuten, höchst bevorzugt 15 bis 30 Minuten lang gehalten wird und wobei der Fettalkohol 8 bis 22 Kohlenstoffatome aufweist;
iv) Ausbilden einer wässrigen Dispersion des Fettalkohols und eines Amidoamins;
Zugeben eines kationischen Tensids zu der wässrigen Dispersion und Mischen; und
Neutralisieren des Amidoamins,
wobei die Temperatur der Mischung des kationischen Tensids in der wässrigen Dispersion bei 56°C bis 67°C, vorzugsweise 10 bis 60 Minuten, bevorzugter 20 bis 40 Minuten, höchst bevorzugt 15 bis 30 Minuten lang gehalten wird.

2. Zusammensetzung nach Anspruch 1, wobei das Polymer ein Acrylatpolymer ist.

3. Zusammensetzung nach irgendeinem vorhergehenden Anspruch, wobei das Polymer ein Methacrylatpolymer ist.

4. Zusammensetzung nach irgendeinem vorhergehenden Anspruch, wobei die Alkylgruppe 16 bis 28 Kohlenstoffatome umfasst.

5. Zusammensetzung nach irgendeinem vorhergehenden Anspruch, umfassend 0,01 bis 5 Gew.-% Polymer.

6. Zusammensetzung nach irgendeinem vorhergehenden Anspruch, wobei das kationische Tensid die Formel N⁺(R)¹(R)²(R)³(R)⁴ aufweist, wobei R¹, R², R³ und R⁴ unabhängig voneinander (C₁- bis C₃₀-) Alkyl oder Benzyl sind.

7. Zusammensetzung nach Anspruch 6, wobei einer, zwei oder drei von R¹, R², R³ und R⁴ unabhängig voneinander (C₄ bis C₃₀)-Alkyl und die andere(n) R¹, R², R³ und R⁴-Gruppe oder Gruppen (C₁ bis C₆-)Alkyl oder Benzyl sind.

8. Zusammensetzung nach irgendeinem vorhergehenden Anspruch, wobei das kationische konditionierende Tensid aus der Gruppe ausgewählt ist, bestehend aus Cetyltrimethylammoniumchlorid, Behenyltrimethylammoniumchlorid und Mischungen davon.

9. Verfahren zum Einsparen von Wasser während eines Konditionierungsverfahrens, umfassend die Schritte des Auftragens einer Zusammensetzung auf Haar, umfassend
a) eine konditionierende Gelphase, umfassend, bezogen auf das Gesamtgewicht der haarkonditionierenden Zusammensetzung,
i) 0,4 bis 8 Gew.-% Fettalkohol mit 8 bis 22 Kohlenstoffatomen,
ii) 0,1 bis 2 Gew.-% eines kationischen Tensids,
b) ein hydrophob modifiziertes anionisches Polymer; und
c) Wasser;
wobei die Zusammensetzung dem mit der konditionierenden Zusammensetzung behandelten Haar eine Ziehmasse von 1 bis 250 g verleiht, wobei die Ziehmasse die Masse darstellt, die erforderlich ist, um eine Haartresse z.B. eines Gewichts von 10 g, einer Länge von 20 cm und einer Breite von 3 cm durch einen Kamm oder eine Bürste zu ziehen, gemessen durch ein erstes Platzieren der Haartresse auf dem Kamm oder der Bürste, so dass 20 cm des Haars an dem verklebten Ende der Tresse hängen bleiben, und dann Gewichtszugaben auf das hängende Ende, bis die Tresse durch den Kamm oder die Bürste fällt;
und wobei die konditionierende Gelphase nach einem Verfahren erhältlich ist, ausgewählt aus Folgendem:
i) Bilden einer Co-Schmelze in einem ersten Gefäß, umfassend Fettalkohol und kationische Komponente und 0,1 bis 15 Gew.-% Wasser, bezogen auf das Gesamtgewicht der Co-Schmelze (A); Zugeben der Co-Schmelze in ein zweites Gefäß, enthaltend Wasser, bei 50 bis 60°C (B); und Mischen,
wobei die Temperatur der Mischung der Co-Schmelze und des Wassers in dem zweiten Gefäß (B) derartig kontrolliert wird, dass sie bei 56 bis 65°C, vorzugsweise bei 58 bis 62°C, bevorzugter bei 60°C, 5 bis 60 min lang, vorzugsweise 10 bis 40 min lang, bevorzugter 15 bis 30 min lang, gehalten wird; wobei der Fettalkohol 8 bis 22 Kohlenstoffatome aufweist;
wobei die kationische Komponente 0,1 bis 70 Gew.-% kationisches Tensid der Formel N⁺R¹R²R³R⁴ umfasst, bevorzugter 30 bis 60 Gew.-%, bezogen auf das Gesamtgewicht der kationischen Komponente; und
wobei R¹, R², R³ und R⁴ unabhängig voneinander (C₁- bis C₃₀-) Alkyl oder Benzyl sind;
ii) Ausbilden einer Co-Schmelze in einem ersten Gefäß, umfassend Fettalkohol und kationische Komponente und 0,1 bis 15 Gew.-% Wasser, bezogen auf das Gesamtgewicht der Co-Schmelze, unabhängig voneinander Zugeben der Co-Schmelze und des Wassers in ein Mischgefäß und Mischen,
wobei die Temperatur der Mischung der Co-Schmelze und des Wassers bei 56 bis 65°C, vorzugsweise bei 58 bis 62°C, bevorzugter bei 60°C, wenn in dem Mischgefäß, 5 bis 60 min, vorzugsweise 10 bis 40 min, bevorzugter 15 bis 30 min lang gehalten wird;
wobei der Fettalkohol 8 bis 22 Kohlenstoffatome aufweist,
wobei die kationische Komponente 0,1 bis 70 Gew.-% kationische Tenside der Formel N⁺R¹R²R³R⁴ umfasst, bevorzugter 30 bis 60 Gew.-%, bezogen auf das Gesamtgewicht der kationischen Komponente, und
wobei R¹, R², R³ und R⁴ unabhängig voneinander (C₁- bis C₃₀-) Alkyl oder Benzyl darstellen;
iii) Ausbilden einer wässrigen isotropen Lösung der kationischen Komponente;
Mischen der wässrigen isotropen Lösung des kationischen Tensids mit geschmolzenem Fettalkohol,
wobei die Temperatur während des Mischens des Fettalkohols mit der isotropen kationischen Tensid-Lösung bei 55°C bis 65°C, vorzugsweise 10 bis 60 Minuten, bevorzugter 20 bis 40 Minuten, höchst bevorzugt 15 bis 30 Minuten lang gehalten wird und wobei der Fettalkohol 8 bis 22 Kohlenstoffatome aufweist;
iv) Ausbilden einer wässrigen Dispersion des Fettalkohols und eines Amidoamins;
Zugeben eines kationischen Tensids zu der wässrigen Dispersion und Mischen; und
Neutralisieren des Amidoamins,
wobei die Temperatur der Mischung des kationischen Tensids in der wässrigen Dispersion bei 56°C bis 67°C, vorzugsweise 10 bis 60 Minuten, bevorzugter 20 bis 40 Minuten, höchst bevorzugt 15 bis 30 Minuten lang gehalten wird.

10. Verfahren, wie in Anspruch 9 beansprucht, wobei die Zusammensetzung nach irgendeinem der Ansprüche 2 bis 8 definiert ist.

11. Verfahren, wie in Anspruch 9 beansprucht, wobei das Haar so lange mit Wasser gespült wird, bis eine konstante Reibung erreicht wird, gemessen unter Verwendung eines Texturanalysators.

12. Verwendung eines hydrophob modifizierten anionischen Polymers in einer Haarbehandlungszusammensetzung, wie in irgendeinem der Ansprüche 1 bis 8 definiert, um während eines Spülschrittes eines konditionierenden Verfahrens Wasser einzusparen, wenn mit einer Vergleichszusammensetzung verglichen wird, die sich von der in den Ansprüchen 1 bis 7 definierten Zusammensetzung darin unterscheidet, dass sie kein hydrophob modifiziertes anionisches Polymer umfasst.

13. Verwendung, wie in Anspruch 12 beansprucht, wobei das Haar so lange gespült wird, bis eine konstante Reibung erreicht wird, gemessen unter Verwendung eines Texturanalysators.

## Revendications

1. Composition de conditionnement des cheveux comprenant :
a) une phase de gel de conditionnement comprenant, en masse totale de la composition de conditionnement des cheveux,
i) de 0,4 à 8 % en masse d'alcool gras ayant de 8 à 22 atomes de carbone,
ii) de 0,1 à 2 % en masse de tensioactif cationique,
b) un polymère anionique hydrophobiquement modifié, qui comprend une modification hydrophobe qui comprend un groupe alkyle comprenant de 6 à 30 atomes de carbone ; et
c) de l'eau,
dans laquelle la composition confère une masse de tirage de 1 à 250 g à des cheveux traités avec la composition de conditionnement, dans laquelle la masse de tirage est la masse nécessaire pour tirer un postiche de cheveux de masse 10 g, longueur 20 cm, et largeur 3 cm à travers un peigne ou une brosse, comme mesuré en disposant dans un premier temps le postiche de cheveux sur le peigne ou la brosse, de sorte que 20 cm de cheveux sont laissés suspendus à l'extrémité collée du postiche, et en ajoutant ensuite des poids à l'extrémité suspendue jusqu'à ce que le postiche tombe à travers le peigne ou la brosse ;
et dans laquelle la phase de gel de conditionnement peut être obtenue à partir d'un procédé choisi parmi les suivants :
i) formation d'une co-fusion dans une première cuve comprenant un alcool gras et un constituant cationique et de 0,1 à 15 % en masse d'eau, en masse totale de la co-fusion (A) ;
addition de la co-fusion dans une seconde cuve contenant de l'eau à de 50 à 60°C (B) ; et mélange,
dans laquelle la température du mélange de la co-fusion et de l'eau dans la seconde cuve (B) est contrôlée de sorte qu'il est maintenu à de 56 à 65°C, de préférence de 58 à 62°C, encore mieux 60°C, pendant de 5 à 60 min, de préférence de 10 à 40 min, encore mieux de 15 à 30 min ;
dans laquelle l'alcool gras présente de 8 à 22 atomes de carbone ;
dans laquelle le constituant cationique comprend de 0,1 à 70 % en masse de tensioactif cationique ayant la formule N⁺R¹R²R³R⁴, encore mieux de 30 à 60 % en masse, en masse totale du constituant cationique ; et
dans laquelle R¹, R², R³ et R⁴ sont indépendamment un alkyle en (C₁ à C₃₀) ou benzyle ;
ii) formation d'une co-fusion dans une première cuve comprenant un alcool gras et un constituant cationique et de 0,1 à 15 % en masse d'eau, en masse totale de la co-fusion, addition indépendante de la co-fusion et de l'eau dans une cuve de mélange et mélange,
dans laquelle la température du mélange de la co-fusion et de l'eau est maintenue à de 56 à 65°C, de préférence de 58 à 62°C, encore mieux 60°C lorsqu'elle est dans la cuve de mélange, pendant de 5 à 60 min, de préférence de 10 à 40 min, encore mieux de 15 à 30 min ;
dans laquelle l'alcool gras comprend de 8 à 22 atomes de carbone,
dans laquelle le constituant cationique comprend de 0,1 à 70 % en masse de tensioactifs cationiques qui présentent la formule N⁺R¹R²R³R⁴, encore mieux de 30 à 60 % en masse, en masse totale du constituant cationique, et
dans laquelle R¹, R², R³ et R⁴ sont indépendamment un alkyle en (C₁ à C₃₀) ou benzyle ;
iii) formation d'une solution isotrope aqueuse de constituant cationique ;
mélange de la solution isotrope aqueuse de tensioactif cationique avec un alcool gras fondu,
dans laquelle la température pendant le mélange de l'alcool gras avec la solution de tensioactif cationique isotrope est maintenue à de 55°C à 65°C, de préférence pendant de 10 à 60 minutes, encore mieux pendant de 20 à 40 minutes, bien mieux encore de 15 à 30 minutes et dans laquelle l'alcool gras présente de 8 à 22 atomes de carbone ;
iv) formation d'une dispersion aqueuse d'alcool gras et d'une amidoamine ; addition d'un tensioactif cationique à la dispersion aqueuse et mélange ; et neutralisation de l'amidoamine,
dans laquelle la température du mélange de tensioactif cationique dans la dispersion aqueuse est maintenue à de 56°C à 67°C, de préférence pendant de 10 à 60 minutes, encore mieux pendant de 20 à 40 minutes, bien mieux encore de 15 à 30 minutes.

2. Composition selon la revendication 1, dans laquelle le polymère est un polymère d'acrylate.

3. Composition selon l'une quelconque des revendications précédentes, dans laquelle le polymère est un polymère de méthacrylate.

4. Composition selon l'une quelconque des revendications précédentes, dans laquelle le groupe alkyle comprend de 16 à 28 atomes de carbone.

5. Composition selon l'une quelconque des revendications précédentes comprenant de 0,01 à 5 % en masse de polymère.

6. Composition selon l'une quelconque des revendications précédentes, dans laquelle le tensioactif cationique présente la formule N⁺(R¹)(R²)(R³)(R⁴), dans laquelle R¹, R², R³ et R⁴ sont indépendamment un alkyle en (C₁ à C₃₀) ou benzyle.

7. Composition selon la revendication 6, dans laquelle un, deux ou trois de R¹, R², R³ et R⁴ sont indépendamment un alkyle en (C₄ à C₃₀) et l'autre groupe ou les autres groupes R¹, R², R³ et R⁴ sont un alkyle en (C₁-C₆) ou benzyle.

8. Composition selon l'une quelconque des revendications précédentes, dans laquelle le tensioactif de conditionnement cationique est choisi dans le groupe consistant en chlorure de cétyltriméthylammonium, chlorure de béhényltriméthylammonium et mélanges de ceux-ci.

9. Procédé d'économie d'eau pendant un procédé de conditionnement, comprenant les étapes d'application aux cheveux d'une composition comprenant
a) une phase de gel de conditionnement comprenant, en masse totale de la composition de conditionnement de cheveux,
i) de 0,4 à 8 % en masse d'alcool gras ayant de 8 à 22 atomes de carbone,
ii) de 0,1 à 2 % en masse de tensioactif cationique,
b) un polymère anionique hydrophobiquement modifié ; et
c) de l'eau,
dans laquelle la composition confère une masse de tirage de 1 à 250 g à des cheveux traités avec la composition de conditionnement, dans laquelle la masse de tirage est la masse exigée pour tirer un postiche de cheveux, par exemple de masse 10 g, longueur 20 cm, et largeur 3 cm à travers un peigne ou une brosse, comme mesuré en disposant dans un premier temps le postiche de cheveux sur le peigne ou la brosse, de sorte que 20 cm de cheveux sont laissés suspendus à l'extrémité collée du postiche, et en ajoutant ensuite des poids à l'extrémité suspendue jusqu'à ce que le postiche tombe à travers le peigne ou la brosse ;
et dans laquelle la phase de gel de conditionnement peut être obtenue à partir d'un procédé choisi parmi les suivants :
i) formation d'une co-fusion dans une première cuve comprenant un alcool gras et un constituant cationique et de 0,1 à 15 % en masse d'eau, en masse totale de la co-fusion (A) ;
addition de la co-fusion dans une seconde cuve contenant de l'eau à de 50 à 60°C (B) ; et mélange,
dans laquelle la température du mélange de la co-fusion et de l'eau dans la seconde cuve (B) est contrôlée de sorte qu'il est maintenu à de 56 à 65°C, de préférence de 58 à 62°C, encore mieux 60°C, pendant de 5 à 60 min, de préférence de 10 à 40 min, encore mieux de 15 à 30 min ;
dans laquelle l'alcool gras présente de 8 à 22 atomes de carbone ;
dans laquelle le constituant cationique comprend de 0,1 à 70 % en masse de tensioactif cationique ayant la formule N⁺R¹R²R³R⁴, encore mieux de 30 à 60 % en masse, en masse totale du constituant cationique ; et
dans laquelle R¹, R², R³ et R⁴ sont indépendamment un alkyle en (C₁ à C₃₀) ou benzyle ;
ii) formation d'une co-fusion dans une première cuve comprenant un alcool gras et un constituant cationique et de 0,1 à 15 % en masse d'eau, en masse totale de la co-fusion, addition indépendante de la co-fusion et de l'eau dans une cuve de mélange et mélange,
dans laquelle la température du mélange de la co-fusion et de l'eau est maintenue à de 56 à 65°C, de préférence de 58 à 62°C, encore mieux 60°C lorsqu'elle est dans la cuve de mélange, pendant de 5 à 60 min, de préférence de 10 à 40 min, encore mieux de 15 à 30 min ;
dans laquelle l'alcool gras comprend de 8 à 22 atomes de carbone,
dans laquelle le constituant cationique comprend de 0,1 à 70 % en masse de tensioactifs cationiques qui présentent la formule N⁺R¹R²R³R⁴, encore mieux de 30 à 60 % en masse, en masse totale du constituant cationique, et
dans laquelle R¹, R², R³ et R⁴ sont indépendamment un alkyle en (C₁ à C₃₀) ou benzyle ;
iii) formation d'une solution isotrope aqueuse de constituant cationique ;
mélange de la solution isotrope aqueuse de tensioactif cationique avec un alcool gras fondu,
dans laquelle la température pendant le mélange de l'alcool gras avec la solution de tensioactif cationique isotrope est maintenue à de 55°C à 65°C, de préférence pendant de 10 à 60 minutes, encore mieux pendant de 20 à 40 minutes, bien mieux encore de 15 à 30 minutes et dans laquelle l'alcool gras présente de 8 à 22 atomes de carbone ;
iv) formation d'une dispersion aqueuse d'alcool gras et d'une amidoamine ; addition d'un tensioactif cationique à la dispersion aqueuse et mélange ; et neutralisation de l'amidoamine,
dans laquelle la température du mélange de tensioactif cationique dans la dispersion aqueuse est maintenue à de 56°C à 67°C, de préférence pendant de 10 à 60 minutes, encore mieux pendant de 20 à 40 minutes, bien mieux encore de 15 à 30 minutes ;
et rinçage des cheveux, comparé à une composition comparative qui est différente de la composition en ce qu'elle ne comprend pas de polymère anionique hydrophobiquement modifié.

10. Procédé selon la revendication 9, dans lequel la composition est comme définie dans l'une quelconque des revendications 2 à 8.

11. Procédé selon la revendication 9, dans lequel les cheveux sont rincés avec de l'eau jusqu'à ce qu'une friction constante soit atteinte comme mesuré en utilisant un dispositif d'analyse de texture.

12. Utilisation d'un polymère anionique hydrophobiquement modifié dans une composition de traitement des cheveux, comme défini par l'une quelconque des revendications 1 à 8, pour économiser de l'eau pendant une étape de rinçage d'un procédé de conditionnement comparé à une composition comparative qui est différente de la composition comme définie dans les revendications 1 à 7 en ce qu'elle ne comprend pas de polymère anionique hydrophobiquement modifié.

13. Utilisation selon la revendication 12, où les cheveux sont rincés jusqu'à ce qu'une friction constante soit atteinte comme mesuré en utilisant un dispositif d'analyse de texture.
